# EUROPEAN PATENT APPLICATION

(11) **EP 4 140 411 A1**
(43) Date of publication of application: **01.03.2023**
(21) Application number: 21792744.1
(22) Date of filing: 22.04.2021
(51) Int. Cl.: A61B 6/00, A61B 6/14, A61B 34/10, A61C 9/00, A61C 8/00

(54) **ORAL IMAGE MARKER DETECTION METHOD, AND ORAL IMAGE MATCHING DEVICE AND METHOD USING SAME**

(30) Priority: 24.04.2020 KR 20200050085; 24.04.2020 KR 20200050086
(71) Applicant: DIO Corporation, Busan 48058 (KR)
(72) Inventor: KIM, Jin Cheol, Busan 48058 (KR); KIM, Jin Baek, Busan 48058 (KR)
(74) Representative: Treeby, Philip David William
(86) International application number: PCT/KR2021/005095
(87) International publication number: WO 2021/215843

(57) **Abstract**

The present invention provides an oral image marker detection method for detecting a marker, which is the reference when an oral scan image and a CT image are matched, the method comprising the steps of: aligning oral scan images in the horizontal plane direction; estimating a marker candidate area from the aligned oral scan images and detecting an undivided marker from the marker candidate area; and dividing the undivided marker to estimate a marker in the undivided marker, and estimating the area from the upper surface of the undivided marker to a certain depth in the lower direction to be the marker.

## Description

### CROSS-REFERENCE TO RELATED APPLICATION

This application claims priority to and the benefit of Korean Patent Application No. 10-2020-0050085, filed on April 24, 2020, and Korean Patent Application No. 10-2020-0050086, filed on April 24, 2020, the disclosures of which are incorporated herein by reference in their entirety.

### [Technical Field]

The present invention relates to an oral image marker detection method, and an oral image matching device and method using the same.

### [Background Art]

In a computer vision, when one scene or object is photographed at different times or viewpoints, images according to different coordinate systems are obtained. Image matching refers to a process of transforming the different images and representing the transformed images in one coordinate system.

Through the image matching, the correspondence relationship between images obtained through different measurement methods may be confirmed.

In dental surgery guide software, an image matching process is generally performed between a computed tomography (CT) image and an oral scan image before entering a dental implant surgery planning step.

Since the images matched through the image matching process become the basis of an implant planning operation of determining a safe and optimal implant placement position by grasping a bone tissue, a nerve tube position and the like, the accuracy of image matching has a very important meaning in proceeding to subsequent procedures.

The image matching method provided by conventional medical software manually inputs a point which is a reference of image matching by an operator, and image matching is performed based on the input point.

According to the conventional image matching method, since the operator roughly determines by the eye and selects a reference point, the result thereof is very inaccurate, and thus, the manual operation process of the operator is inevitably performed after image matching. That is, the operator changes the position of a point or reselects a point to correct a matching result. As described above, according to prior art, due to a continuous repetitive process of matching and correction, the operator consumes a lot of time for image matching, and there is a problem in that it is difficult to obtain a result that satisfies the consumed time.

As another conventional method, there may be a method of acquiring an image including markers to be utilized as intraoral matching references, and matching an image acquired from a heterogeneous image capturing device based on the markers in the image.

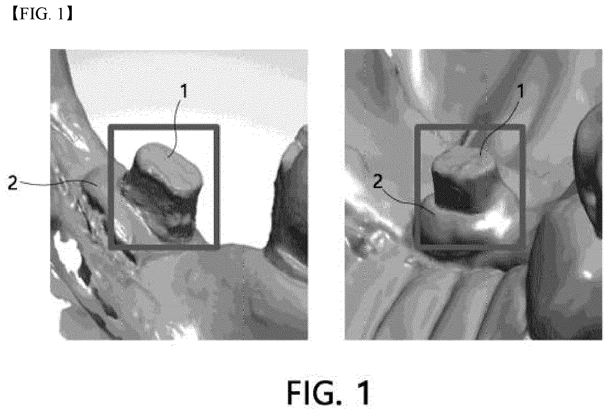

FIG. 1 is a diagram illustrating markers attached to the inside of an oral cavity for image matching.

Referring to FIG. 1, a plurality of markers 1 are attached to predetermined positions in the oral cavity of a subject by using an adhesive (e.g., resin). Afterwards, when a certain time elapses, an adhesive layer 2 is formed between the marker 1 and the gum. In this case, since the amount and shape of the adhesive are different for each attachment position of the marker 1, the shape of the adhesive layer 2 becomes non-uniform.

Meanwhile, in terms of detecting the marker 1 in the oral image for image matching, since the marker 1 and the adhesive layer 2 are integrally formed, the marker 1 and the adhesive layer 2 may be detected together. However, the non-uniform adhesive layer 2 becomes an obstacle to matching during image matching, and causes a problem of lowering the matching accuracy during image matching.

### [Disclosure]

### [Technical Problem]

An object of the present invention is to provide an oral image matching device and method for detecting a marker in an oral image, which remove factors that interfere with matching in the detected marker and automatically perform image matching with high accuracy by using the detected marker.

### [Technical Solution]

In order to solve the aforementioned problems, the present invention provides an oral image marker detection method for detecting a marker, which is the reference when an oral scan image and a CT image are matched, including the steps of aligning oral scan images in the horizontal plane direction; estimating a marker candidate area from the aligned oral scan images and detecting an undivided marker from the marker candidate area; and dividing the undivided marker to estimate a marker in the undivided marker, and estimating the area from the upper surface of the undivided marker to a certain depth in the lower direction to be the marker.

In addition, the present invention provides a device for matching an oral scan image and a CT image by using a marker, including an image alignment unit for aligning oral scan images in the horizontal plane direction; an undivided market detection unit for estimating a marker candidate area from the aligned oral scan images and detecting an undivided marker from the marker candidate area; a marker estimation unit for dividing the undivided marker to estimate a marker in the undivided marker, and estimating the area from the upper surface of the undivided marker to a certain depth in the lower direction to be the marker; a marker detection unit for detecting the marker in the CT image by using the volume information of the marker; and an image matching unit for matching the oral scan image and the CT image by using the marker.

The present invention provides an oral image matching method for matching an oral scan image and a CT image, including the steps of: respectively dividing a first marker and a first tooth area in the oral scan image; generating a first matching reference point by integrating the first marker and the first tooth area; dividing a second marker and a second tooth area in the CT image; generating a second matching reference point by integrating the second marker and the second tooth area; and matching the oral scan image and the CT image based on the first and second matching reference points.

In addition, the present invention provides an oral image matching device for matching an oral scan image and a CT image, including a first marker dividing unit for dividing a first marker in the oral scan image; a first tooth area dividing unit for dividing a first tooth area in the oral scan image; a first matching reference point generating unit for generating a first matching reference point by integrating the first marker and the first tooth area; a second marker dividing unit for dividing a second marker in the CT image; a second tooth area dividing unit for dividing a second tooth area in the CT image; a second matching reference point generating unit for generating a second matching reference point by integrating the second marker and the second tooth area; and an image matching unit for matching the oral scan image and the CT image based on the first and second matching reference points.

### [Advantageous Effects]

According to the present invention, the user's convenience is improved by detecting a marker in the oral image, removing factors that interfere with matching in the detected marker, and automatically performing image matching with high accuracy by using the detected marker, and accordingly, it has the effects of reducing the time required for implant planning and improving the accuracy of implant planning.

### [Description of Drawings]

FIG. 1 is a diagram illustrating markers attached to the inside of an oral cavity for image matching.
FIG. 2 is a schematic block diagram of the oral image matching device according to an exemplary embodiment of the present invention.
FIG. 3 is a detailed block diagram of a control unit according to a first example of the present invention.
FIG. 4 is a flowchart of the oral image matching method according to a first example of the present invention.
FIG. 5 is a diagram illustrating a marker candidate area estimated from an oral scan image.
FIG. 6 is a diagram illustrating undivided markers detected in the marker candidate area of FIG. 5.
FIG. 7 is a diagram illustrating markers included in the undivided markers of FIG. 6 and an adhesive layer separately.
FIG. 8 is an enlarged view of the undivided markers of FIG. 7.
FIG. 9 is a diagram illustrating markers estimated from an oral scan image.
FIG. 10 is a perspective view illustrating markers according to a first example of the present invention.
FIG. 11 is a diagram illustrating a CT image generated by photographing an oral cavity to which the markers of FIG. 10 is attached.
FIG. 12 is a diagram illustrating markers detected in the CT image of FIG. 11.
FIG. 13 is a diagram illustrating matching of an oral scan image and a CT image by using the oral image matching method according to the first example of the present invention.
FIG. 14 is a detailed block diagram of a control unit according to a second example of the present invention.
FIG. 15 is a flowchart of the oral image matching method according to a second example of the present invention.
FIG. 16 is a diagram illustrating an oral scan image generated by photographing an oral cavity to which markers are attached.
FIG. 17 is an enlarged view of undivided markers.
FIG. 18 is a diagram illustrating markers divided from the undivided markers of FIG. 17.
FIG. 19 is a diagram for describing the method of calculating the curvature of a plurality of meshes included in an oral scan image.
FIG. 20 is a diagram illustrating a tooth area divided in an oral scan image based on the curvatures of a plurality of meshes calculated in FIG. 19.
FIG. 21 is a diagram illustrating a first matching reference point in an oral scan image.
FIG. 22 is a diagram illustrating a CT image generated by photographing an oral cavity to which markers are attached.
FIG. 23 is a diagram illustrating markers detected in the CT image of FIG. 22.
FIG. 24 is a diagram illustrating a divided tooth area in a CT image.
FIG. 25 is a diagram illustrating a second matching reference point in a CT image.
FIG. 26 is a diagram illustrating the matching of an oral scan image and a CT image by the oral image matching method according to the second example of the present invention.

### [Modes of the Invention]

The objects and means of the present invention and the effects therefrom will become more apparent from the following detailed description in conjunction with the accompanying drawings, and accordingly, the present invention will be easily practiced by those of ordinary skill in the art to which the present invention pertains. Further, in terms of describing the present invention, if it is determined that well-known technologies related to the present invention would obscure the gist of present invention due to unnecessary detail, the detailed description thereof will be omitted.

Hereinafter, preferred exemplary embodiments according to the present invention will be described in detail with reference to the accompanying drawings.

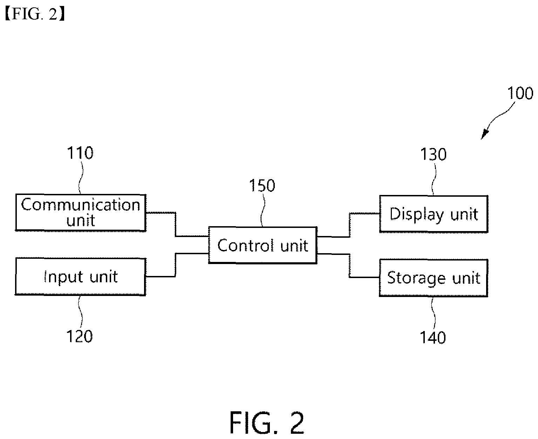

FIG. 2 is a schematic block diagram of the oral image matching device according to an exemplary embodiment of the present invention.

The oral image matching device 100 according to an exemplary embodiment of the present invention is an electronic device executable by a computer program, and it is a device of matching the oral scan image and CT image of a subject by using at least one of a marker and a tooth area as a matching reference point. Herein, the tooth area may be one individual tooth or a form in which a plurality of adjacent individual teeth are combined, and the number of markers may be at least one, but the present invention is not limited thereto. In addition, the marker may be attached to the missing tooth area.

The oral scan image may provide information about the shape of the crown portion of a tooth exposed to the outside and the shape of the gum around the tooth as 3D images. In this case, the oral scan image may be obtained by directly scanning the inside of the subject's oral cavity through an oral scanner or the like, or scanning an impression model that mimics the inside of the subject's oral cavity with an intaglio or a plaster model created by embossing the impression model, and the scan image of the impression model may be inverted and used as an oral scan image.

As a 3D image, the CT image may provide information on not only the crown, but also the shape of the root and alveolar bone, as well as the bone density of the crown, root and alveolar bone. In this case, the CT image may be obtained by performing a CT scan of the inside of the subject's oral cavity.

The matching image, in which the oral scan image and CT image are matched, becomes the basic image of an implant planning operation that determines the safe and optimal implant placement position by identifying the bone tissue and neural tube position of the subject.

Referring to FIG. 2, the oral image matching device 100 according to an exemplary embodiment of the present invention may include a communication unit 110, an input unit 120, a display unit 130, a storage unit 140 and a control unit 150.

The communication unit 110 is a configuration for communicating with an external device such as an image acquisition device (not illustrated) and a server (not illustrated), and may receive image data. For example, the communication unit 110 may perform wireless communication such as 5^{th} generation communication (5G), long term evolution-advanced (LTE-A), long term evolution (LTE), Bluetooth, Bluetooth low energy (BLE), near field communication (NFC) and the like, and may perform wired communication such as cable communication and the like. In this case, the image data may include oral scan image data and CT image data.

The input unit 120 generates input data in response to a user's input, and may include at least one input means. For example, the input unit 120 may include a keyboard, a keypad, a dome switch, a touch panel, a touch key, a mouse, a menu button and the like.

The display unit 130 displays display data according to the operation of the oral image matching device 100. The display data may include image data. For example, the display unit 130 may include a liquid crystal display (LCD), a light emitting diode (LED) display, an organic light emitting diode (OLED) display, and a micro-electromechanical system (MEMS) display and an electronic paper display. In addition, the display unit 130 may be implemented as a touch screen in combination with the input unit 120.

The storage unit 140 stores various information and programs necessary for the operation of the oral image matching device 100. For example, the storage unit 140 may store image data received from an image acquisition device or the like, algorithms related to image alignment, marker and tooth area estimation and detection, and an image matching method according to an exemplary embodiment of the present invention, which will be described below. In addition, the storage unit 140 may store a learning model obtained by learning a plurality of learning images by a deep learning method in order to detect a marker and a tooth area.

The control unit 150 detects a marker and a tooth area from oral image data received from an image acquisition device or server, or pre-stored in the storage unit 140, and performs matching the oral image data using the detected marker and the tooth area as reference points. To this end, the control unit 150 may receive the oral image data from the image acquisition device or the server and store it in the storage unit 140. In addition, the control unit 150 may control the operations of the communication unit 110, the input unit 120, the display unit 130 and the storage unit 140.

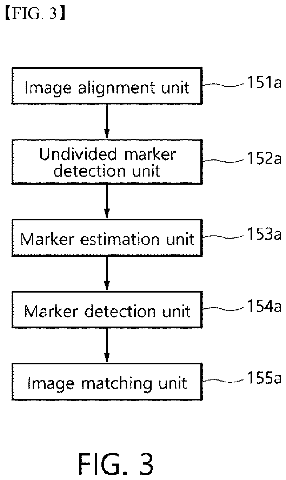

FIG. 3 is a detailed block diagram of a control unit according to a first example of the present invention.

Referring to FIG. 3, the control unit 150 may include an image alignment unit 151a, an undivided marker detection unit 152a, a marker estimation unit 153a, a marker detection unit 154a and an image matching unit 155a.

First, the image acquisition device acquires an oral scan image and a CT image, respectively, by photographing the inside of the oral cavity to which the marker is attached. In this case, the acquired oral scan image and CT image may be directly transmitted to the image alignment unit 151a or may be stored in the storage unit 140 and then transmitted to the image alignment unit 151a.

The image alignment unit 151a aligns oral scan images in the horizontal plane direction such that the marker faces upward in the oral scan image.

The undivided marker detection unit 152a estimates a marker candidate area from the oral scan image aligned by the image alignment unit 151a, and detects an undivided marker from the marker candidate area.

The marker estimation unit 153a divides the undivided marker detected by the marker candidate detection unit 152a up and down, and estimates the marker from the undivided marker, and it estimates the area from the upper surface of the undivided marker to a predetermined depth in the lower direction as the marker, and estimates the other area as an adhesive layer.

The marker detection unit 154a detects a marker by using the volume information of the marker in the CT image.

The image matching unit 155a matches the oral scan image and the CT image by using the markers estimated and detected by the marker estimation unit 153a and the marker detection unit 154a, respectively.

As described above, according to the oral image matching device 100 of the present invention, the marker is detected in the oral image, the adhesive layer that interferes with the matching is removed from the detected marker, and the detected marker is used as a matching reference point to automatically perform image matching with high accuracy, and thus, the user's convenience is improved, and accordingly, it has the effects of reducing the time required for implant planning and improving the accuracy of implant planning.

Hereinafter, the method for detecting a marker of an oral image and the method for matching an oral image according to an exemplary embodiment of the present invention that are controlled by the control unit 150 will be described.

The method for detecting a marker of an oral image and the method for matching an oral image according to a second example of the present invention is characterized in that the oral images of the subject, particularly, the edentulous oral images are matched. Herein, since there is no tooth that can be used as a reference point for matching in the edentulous jaw, oral images are taken while markers are attached to the subject's gum, and the oral images are matched by using these marker as reference points.

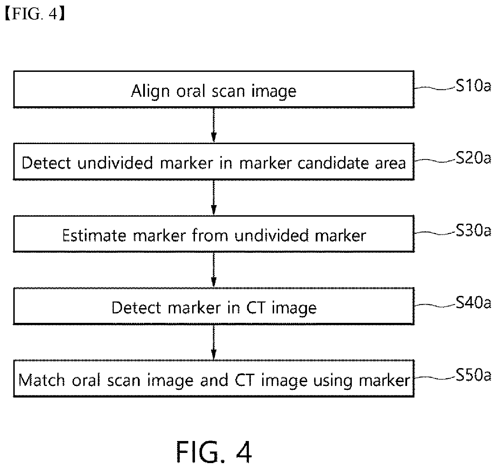

FIG. 4 is a flowchart of the oral image matching method according to a second example of the present invention.

First, markers are attached to a plurality of positions (e.g., gum) in the oral cavity of a subject by using an adhesive such as resin. In this case, when a predetermined time elapses, an adhesive layer is formed between the marker and the gum. Thereafter, a heterogeneous image acquisition device captures the inside of the oral cavity to which the markers are attached to acquire an oral scan image and a CT image, respectively.

Referring to FIG. 4, the oral image matching method according to an exemplary embodiment of the present invention is a method of matching an oral scan image and a CT image by using markers, and may include the steps of aligning oral scan images (S10a), detecting an undivided marker in ta marker candidate area (S20a), estimating a marker from the undivided marker (S30a), detecting the marker in the CT image (S40a) and matching the oral scan image and the CT image by using the marker (S50a)

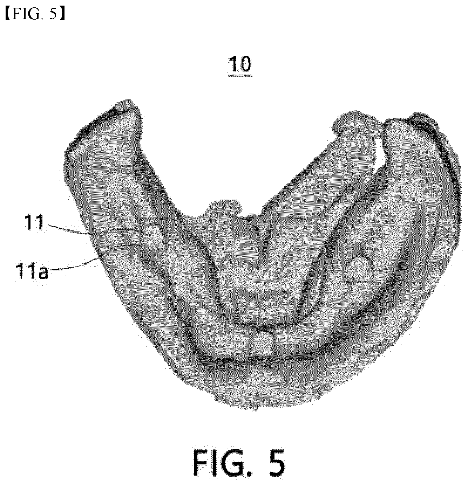

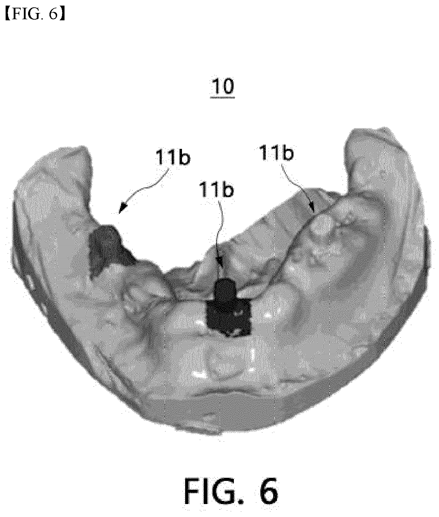

FIG. 5 is a diagram illustrating a marker candidate area estimated from an oral scan image, and FIG. 6 is a diagram illustrating undivided markers detected in the marker candidate area of FIG. 5.

Hereinafter, the marker 11 and the adhesive layer 12 will be collectively referred to as an undivided marker 11b.

Referring to FIGS. 4 to 6, the oral scan image 10 is aligned in the horizontal plane direction such that the marker 11 faces upward in the oral scan image 10 (S10a). Then, the candidate marker region 11a is estimated from the aligned oral scan image 10, and the undivided marker 11b is detected from the candidate marker region 11a (S20a).

The undivided marker 11b may be detected by using a learning model. That is, by using a deep learning method (e.g., YOLO (You Only Look Once) algorithm), a plurality of learning images to which the marker 11 is attached may be learned to generate a learning model, and the oral scan image 10 of a subject may be input to the generated learning model so as to detect the undivided marker 11b from the oral scan image 10. Herein, the number, size and coordinate information of the undivided markers 11b may be obtained.

Since the undivided marker 11b includes not only the marker 11 but also the non-uniform adhesive layer 12, that is, since the marker 11 and the adhesive layer 12 are integrally formed, the shape thereof is non-uniform. Accordingly, in order to improve the matching accuracy, it is necessary to divide the marker 11 from the undivided marker 11b.

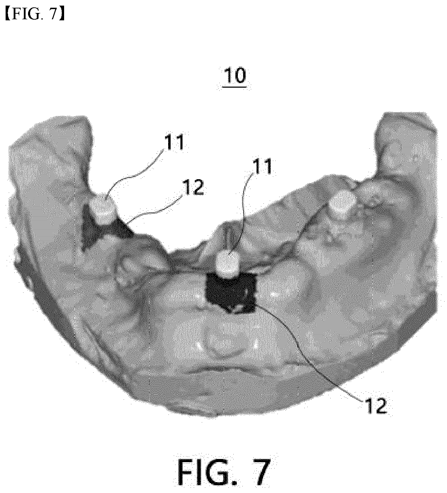

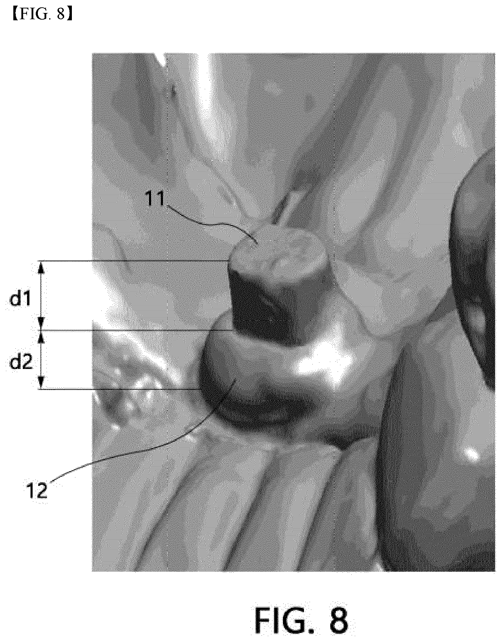

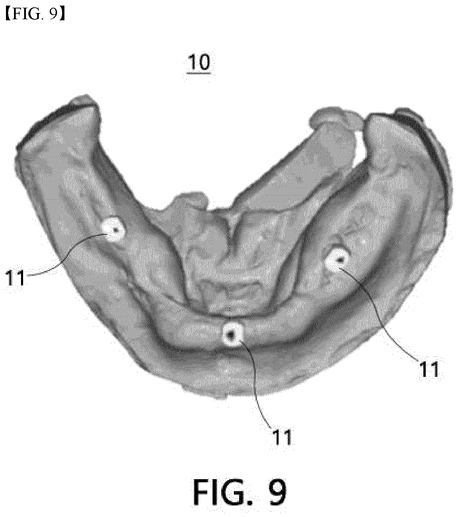

FIG. 7 is a diagram illustrating markers included in the undivided markers of FIG. 6 and an adhesive layer separately, FIG. 8 is an enlarged view of the undivided markers of FIG. 7, and FIG. 9 is a diagram illustrating markers estimated from an oral scan image.

Referring to FIGS. 7 to 9, the area from the upper surface of the undivided marker 11b to a predetermined depth (d1) in the lower direction is estimated as a marker 11 (S30a). In addition, the area exceeding the predetermined depth (d1) (*e.g.,* 5 mm) in the undivided marker 11b is estimated as an adhesive layer 12 interposed between the marker 11 and the gum. That is, the area from the lower surface of the undivided marker 11b to a predetermined height (d2) in the upper direction is estimated as the adhesive layer 12. Accordingly, the marker 11 may be divided from the undivided marker 11b.

Meanwhile, unlike the drawings, by aligning the oral scan image 10 in the horizontal plane direction such that the marker 11 faces downward in the oral scan image 10, it is also possible to detect the undivided marker 11b and divide the marker 11 from the undivided marker 11b. In this case, the area from the lower surface of the undivided marker 11b to a predetermined height (d1) in the upper direction is estimated as the marker 11, and the area exceeding the predetermined height (d1) (*e.g.,* 5 mm) from the undivided marker 11b is estimated as the adhesive layer 12 interposed between the marker 11 and the gum.

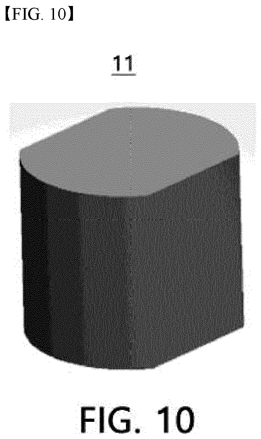

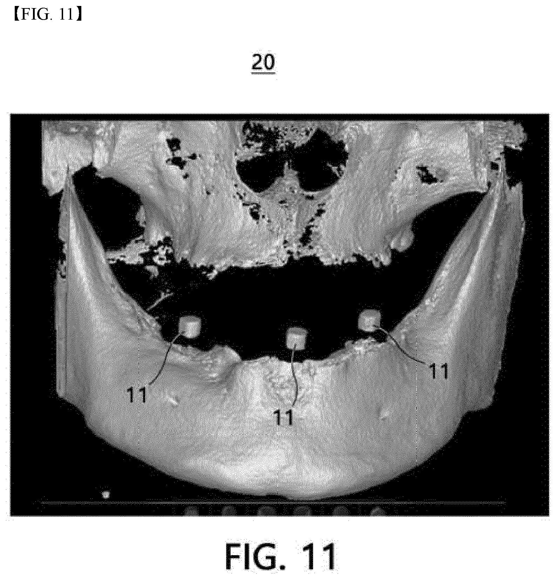

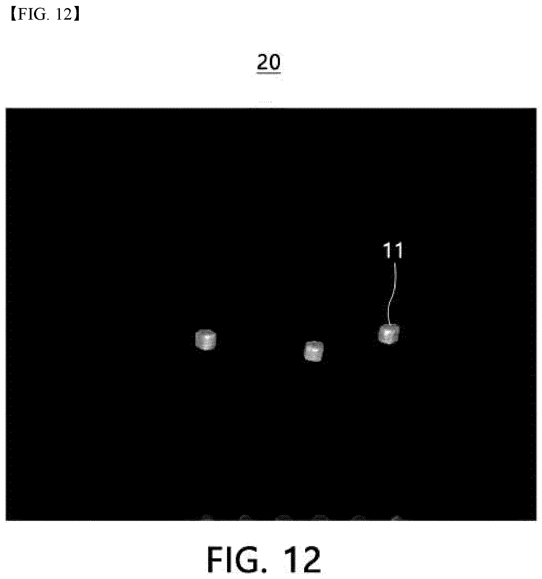

FIG. 10 is a perspective view illustrating markers according to a first example of the present invention, FIG. 11 is a diagram illustrating a CT image generated by photographing an oral cavity to which the markers of FIG. 10 is attached, and FIG. 12 is a diagram illustrating markers detected in the CT image of FIG. 11.

Referring to FIGS. 10 to 12, the marker 11 may have a three-dimensional shape (*e.g.,* a rectangular parallelepiped) having a predetermined volume. Herein, the volume of the rectangular parallelepiped may be defined as a product of width, length and height (*e.g.,* 4 mm × 5 mm × 5 mm). In addition, at least three or more markers 11 may be attached to predetermined positions in the oral cavity (e.g., gum), but the present invention is not limited thereto.

Meanwhile, referring to FIG. 11, the marker 11 may be made of a radiopaque material (*e.g*., alumina), and the adhesive layer 12 may be made of a radioactive material. Accordingly, in the CT image 20, the marker 11 is displayed by including the crown, the root and the alveolar bone, but the adhesive layer 12 is not displayed. Accordingly, in the CT image 20, it is not necessary to divide the marker 11 and the adhesive layer 12 as in the oral scan image 10. However, it is difficult to accurately detect the marker 11 because it is not easy to distinguish between the crown, tooth root and alveolar bone and the marker 11.

In order to accurately detect the marker 11 in the CT image 20 as described above, the marker 11 is detected in the CT image 20 by using the volume information of the marker 11 (S40a). Specifically, a mask is created in a shape corresponding to the marker 11 by using the volume information of the marker 11. Then, the marker 11 is searched for in the CT image 20 by using the mask, and the marker 11 having a shape corresponding to the mask is detected. Herein, the number, size and coordinate information of the markers 11 may be obtained.

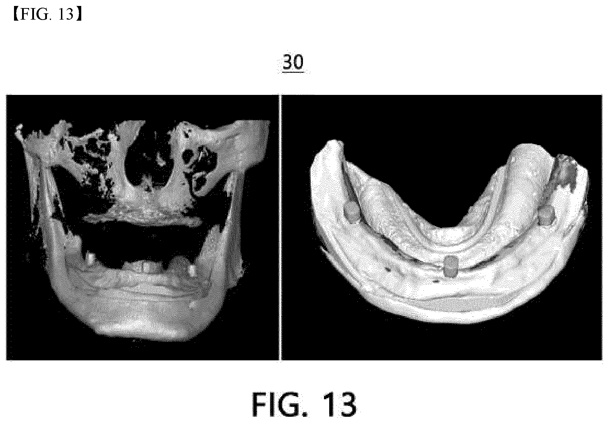

FIG. 13 is a diagram illustrating matching of an oral scan image and a CT image by using the oral image matching method according to the first example of the present invention.

Referring to FIG. 13, the oral scan image 10 and the CT image 20 are automatically matched by using the marker 11 estimated and detected from the oral scan image 10 and the CT image 20 as a matching reference point. (S50a).

As such, according to the oral image matching method of the present invention, the marker 11 is detected in the oral image, and the adhesive layer 12 that interferes with the matching is removed, and the detected marker 11 is used as a matching reference point with high accuracy to automatically perform image matching with high accuracy, and thus, the user's convenience is improved, and accordingly, it has the effects of reducing the time required for implant planning and improving the accuracy of implant planning.

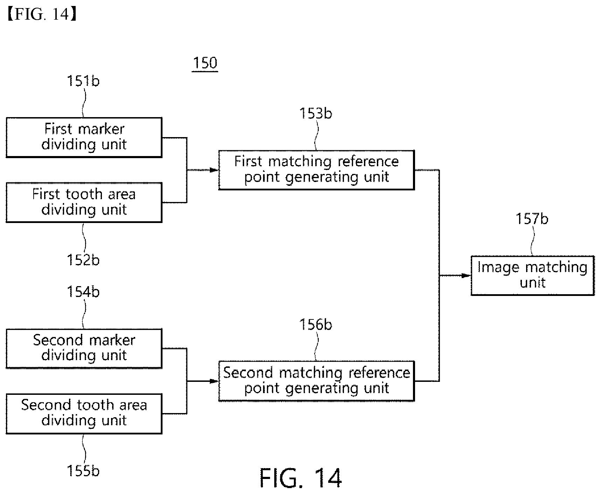

FIG. 14 is a detailed block diagram of a control unit according to a second example of the present invention.

Referring to FIG. 14, the control unit 150 may include a first marker dividing unit 151b, a first tooth area dividing unit 152b, a first matching reference point generating unit 153b, a second marker dividing unit 154b, a second tooth area dividing unit 155b, a second matching reference point generating unit 156b and an image matching unit 157b.

First, the image acquisition device acquires an oral scan image and a CT image, respectively, by photographing the inside of the oral cavity to which the marker is attached. In this case, the acquired oral scan image and CT image may be directly transmitted to the image alignment unit 151b or may be stored in the storage unit 140 and then transmitted to the image alignment unit 151b.

The first marker dividing unit 151b divides a first marker in the oral scan image. Specifically, the first marker dividing unit 151b aligns oral scan images in the horizontal plane direction, estimates a marker candidate area from the aligned oral scan images, and detects an undivided marker in the marker candidate area. Then, the first marker is estimated from the undivided marker by dividing the undivided marker, and the area from the upper surface of the undivided marker to a predetermined depth in the lower direction is estimated as the first marker.

The first tooth area dividing unit 152b divides the first tooth area in the oral scan image. Specifically, the first tooth area dividing unit 152b calculates curvatures of a plurality of meshes included in the oral scan image, and calculates a boundary between the first tooth area and the gum region in the oral scan image based on the curvatures of the plurality of meshes. Then, the first tooth area is divided in the oral scan image based on the boundary between the first tooth area and the gum area.

The first matching reference point generating unit 153b generates a first matching reference point by integrating the first marker and the first tooth area.

The second marker dividing unit 154b divides the second marker in the CT image. Specifically, the second marker dividing unit 154b generates a mask in a shape corresponding to the second marker by using the volume information of the second marker, and searches for the second marker in the CT image by using the mask to divide the second marker.

The second tooth area dividing unit 155b divides the second tooth area in the CT image. Specifically, the second tooth area dividing unit 155b generates a second learning model by learning a second learning image based on Hounsfield unit (HU) values of a plurality of voxels included in the CT image, and divides a second tooth area in the CT image based on the second learning image by inputting the oral scan image to the second learning model.

The second matching reference point generating unit 156b generates a second matching reference point by integrating the second marker and the second tooth area. In addition, the image matching unit 157b matches the oral scan image and the CT image based on the first and second matching reference points.

As described above, according to the oral image matching device 100 of the present invention, the marker and the tooth area are divided in the oral image, and factors that interfere with matching are removed from the marker, and image matching is automatically performed with high accuracy by using the divided marker and the tooth area, and thus, the user's convenience is improved, and as a result, it has the effects of reducing the time required for implant planning and improving the accuracy of implant planning.

Hereinafter, the oral image matching method of oral images according to a second example of the present invention controlled by the control unit 150 will be described.

In the oral image matching method according to the second example of the present invention, oral images are taken while a marker is attached to the gum of the subject, and the oral images are matched by using the marker and the tooth area as reference points.

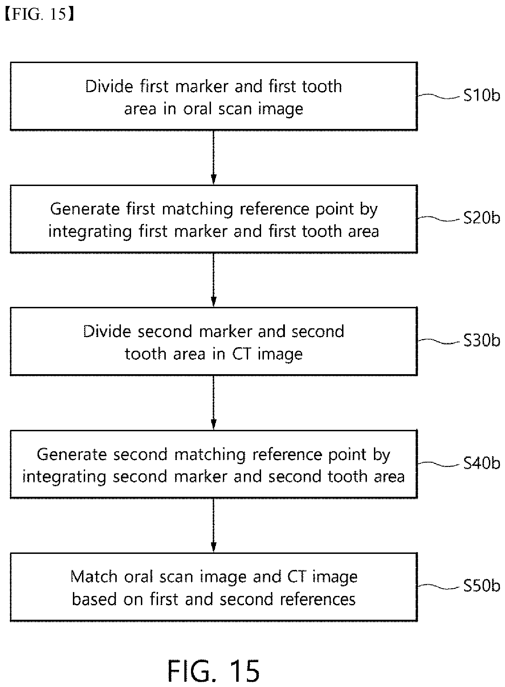

FIG. 15 is a flowchart of the oral image matching method according to a second example of the present invention.

First, markers are attached to a plurality of positions (e.g., gum), particularly in the area of a missing tooth, in the subject's oral cavity by using an adhesive such as a resin. In this case, when a predetermined time elapses, an adhesive layer is formed between the marker and the gum. Thereafter, a heterogeneous image acquisition device captures the inside of the oral cavity to which the marker is attached to acquire an oral scan image and a CT image, respectively.

Referring to FIG. 15, the oral image matching method according to the second example of the present invention is a method of matching an oral scan image and a CT image by using a marker and a tooth area, it may include the steps of dividing a first marker and a first tooth area in the oral scan image (S10b), integrating the first marker and the first tooth area to generate a first registration reference point (S20b), dividing a second marker and a second tooth area in a CT image (S30b), integrating the second marker and the second tooth region to generate a second registration reference point (S40b), and matching the oral scan image and the CT image based on the first and second registration reference points (S50b).

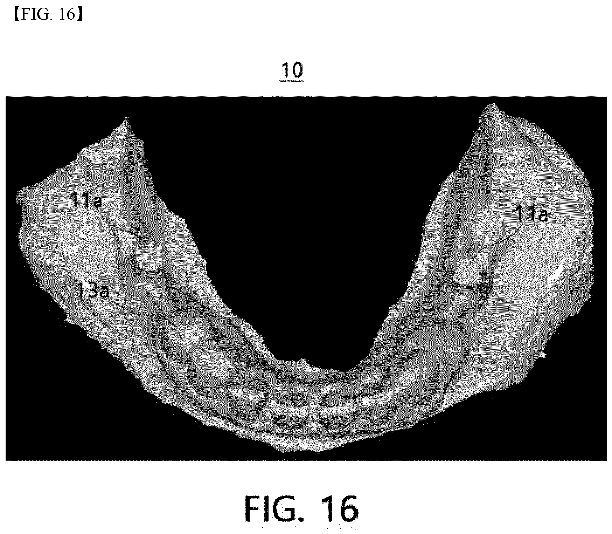

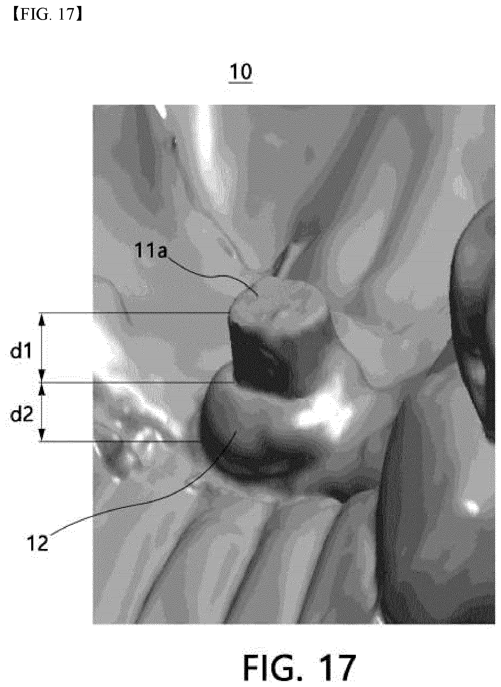

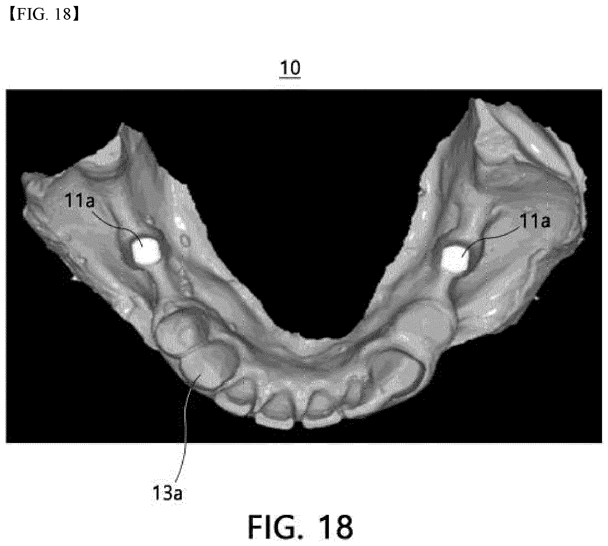

FIG. 16 is a diagram illustrating an oral scan image generated by photographing an oral cavity to which markers are attached, FIG. 17 is an enlarged view of undivided markers, FIG. 18 is a diagram illustrating markers divided from the undivided markers of FIG. 17.

Hereinafter, the method of dividing the first marker 11a in the oral scan image 10 will be described with reference to FIGS. 16 to 18. Hereinafter, the first marker 11a and the adhesive layer 12 will be collectively referred to as an undivided marker.

The oral scan image 10 is aligned in the horizontal plane direction such that the marker 11a faces upward in the oral scan image 10. Then, a marker candidate area is estimated from the aligned oral scan image 10, and an undivided marker is detected from the marker candidate area.

The undivided marker may be detected by using a learning model. That is, by using a deep learning method (e.g., You Only Look Once (YOLO) algorithm), a first learning model may be generated by learning a plurality of learning images to which a first marker 11a is attached, and the undivided marker may be detected from the oral scan image 10 by inputting the oral scan image 10 of the subject to the generated first learning model. Herein, the number, size and coordinate information of undivided markers may be obtained.

Since the undivided marker includes the non-uniform adhesive layer 12 as well as the first marker 11a, that is, since the first marker 11 and the adhesive layer 12 are integrally formed, the shape thereof is non-uniform. Accordingly, in order to improve the matching accuracy, it is necessary to divide the first marker 11a from the undivided marker.

Referring to FIG. 17, the area from the top surface of the undivided marker to a predetermined depth (d 1) in the lower direction is estimated as a first marker 11a. In addition, the area exceeding the predetermined depth (d1) (*e.g.*, 5 mm) in the undivided marker is estimated as an adhesive layer 12 interposed between the first marker 11a and the gum. That is, the area from the lower surface of the undivided marker to a predetermined height (d2) in the upper direction is estimated as the adhesive layer 12. Accordingly, the marker 11 may be divided from the undivided marker 11b (S10b).

Meanwhile, unlike the drawings, by aligning the oral scan image 10 in the horizontal plane direction such that the first marker 11a in the oral scan image 10 faces downward, the undivided marker may be detected and the first marker may be divided from the undivided marker 11a. In this case, the area from the lower surface of the undivided marker to a predetermined height (d1) in the upper direction is estimated as a first marker 11a, and the area exceeding the predetermined height (d1) (*e.g*., 5 mm) in the undivided marker is estimated as an adhesive layer 12 interposed between the first marker 11a and the gum.

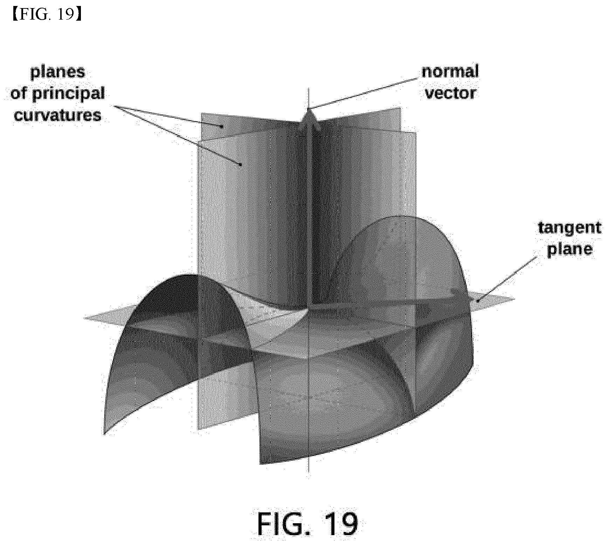

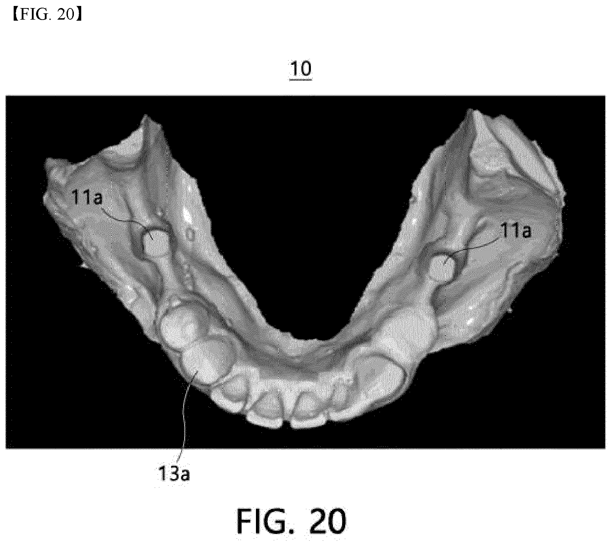

FIG. 19 is a diagram for describing the method of calculating the curvature of a plurality of meshes included in an oral scan image, and FIG. 20 is a diagram illustrating a tooth area divided in an oral scan image based on the curvatures of a plurality of meshes calculated in FIG. 19.

Hereinafter, the method of dividing the first tooth area in the oral scan image will be described with reference to FIGS. 19 and 20.

Curvatures of a plurality of meshes included in the oral scan image 10 are calculated, and a boundary between the first tooth area 13a and the gum region is calculated in the oral scan image 10 based on the curvatures of the plurality of meshes.

Specifically, a plane that includes a normal vector and intersects the mesh surface is drawn in a vertex shared by adjacent meshes, and then the plane is rotated based on the normal vector and continuously intersects the mesh surface. In this case, a number of curves centered on the vertex may be obtained on the mesh surface, and the curvature may be calculated by using the angle between these curves and the tangent plane of the vertex. In addition, the largest value among these curvatures may be defined as the maximum curvature, and the smallest value may be defined as the minimum curvature. Meanwhile, a plane including two curves having the largest maximum curvature and the smallest minimum curvature at the vertex among the calculated curvatures may be defined as a plane of principal curvatures.

Herein, the Gaussian curvature is defined as the product of the maximum curvature and the minimum curvature, and the average curvature is defined as the average of the maximum curvature and the minimum curvature.

Meshes, in which at least one of the Gaussian curvature and the average curvature calculated in this way is equal to or greater than the reference curvature, are selected, and a vertex shared by the selected meshes is determined as a boundary point.

In addition, the boundary points are sequentially expanded (dilation) and reduced (erosion) by using a morphology calculation algorithm to connect adjacent boundary points. Herein, the morphology calculation algorithm is an algorithm for expanding and reducing an area, and is generally used for connecting or disconnecting adjacent points.

In this way, when the boundary points are expanded and then reduced through the reduction process, the boundary between the teeth and the gum area may be further improved, because only the connection between adjacent boundary points is performed while maintaining the thickness of the existing boundary point component.

Accordingly, the first tooth area 13a may be divided in the oral scan image 10 based on the boundary between the first tooth area 13a and the gum area (S10b).

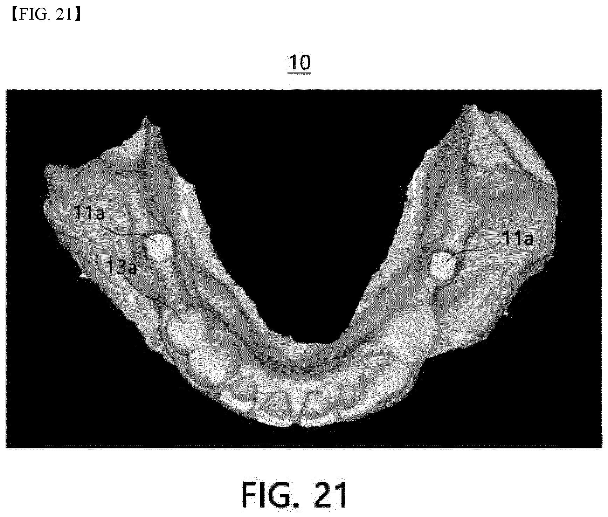

FIG. 21 is a diagram illustrating a first matching reference point in an oral scan image.

As illustrated in FIG. 21, the first marker 11a and the first tooth area 13a that are respectively divided in the oral scan image 10 are integrated to generate a first matching reference point that serves as a reference for matching the oral scan image 10 and the CT image (S20b).

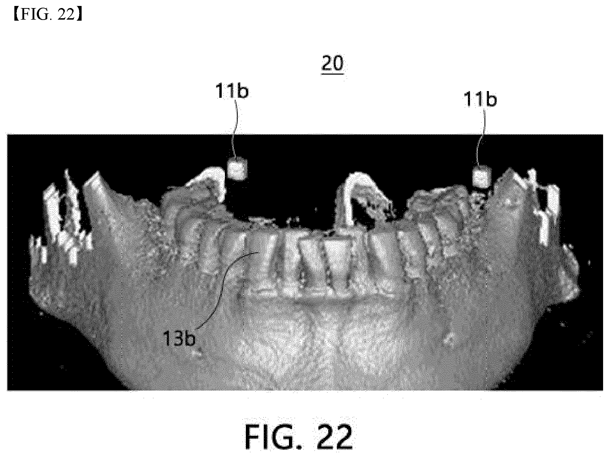

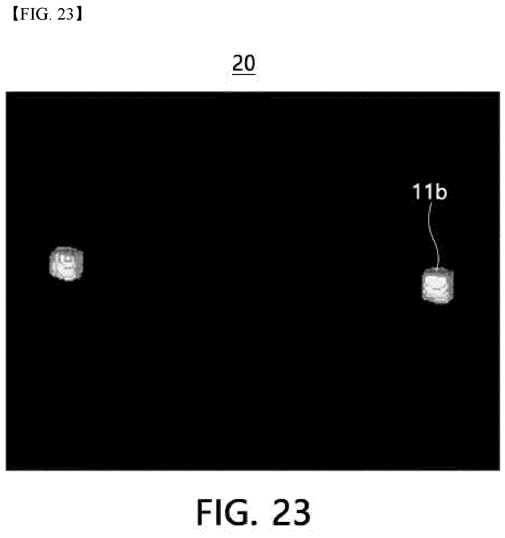

FIG. 22 is a diagram illustrating a CT image generated by photographing an oral cavity to which markers are attached, and FIG. 23 is a diagram illustrating markers detected in the CT image of FIG. 22.

Referring to FIGS. 22 and 23, the second marker 11b may have a three-dimensional shape (e.g., a rectangular parallelepiped) having a predetermined volume. Herein, the volume of the rectangular parallelepiped may be defined as a product of width, length and height (e.g., 4 mm × 5 mm × 5 mm). In addition, at least one second marker 11b may be attached to a predetermined position in the oral cavity (e.g., gum), but the present invention is not limited thereto.

Meanwhile, referring to FIG. 22, the second marker 11b may be made of a radiopaque material (*e.g*., alumina), and the adhesive layer 12 may be made of a radioactive material. Accordingly, in the CT image 20, the second marker 11b is displayed by including the crown, root, and alveolar bone, but the adhesive layer 12 is not displayed. Accordingly, in the CT image 20, it is not necessary to divide the second marker 11b and the adhesive layer 12 as in the oral scan image 10. However, it is difficult to accurately detect the second marker 11b because it is not easy to distinguish between the crown, tooth root and alveolar bone and the second marker 11b.

In order to accurately detect the second marker 11b in the CT image 20 as described above, the second marker 11b is detected and divided in the CT image 20 by using the volume information of the second marker 11b (S30b). Specifically, a mask is created in a shape corresponding to the second marker 11b by using the volume information of the second marker 11b. Then, the second marker 11b is searched for in the CT image 20 by using the mask, and the second marker 11b having a shape corresponding to the mask is detected and divided. Herein, the number, size and coordinate information of the second markers 11b may be obtained.

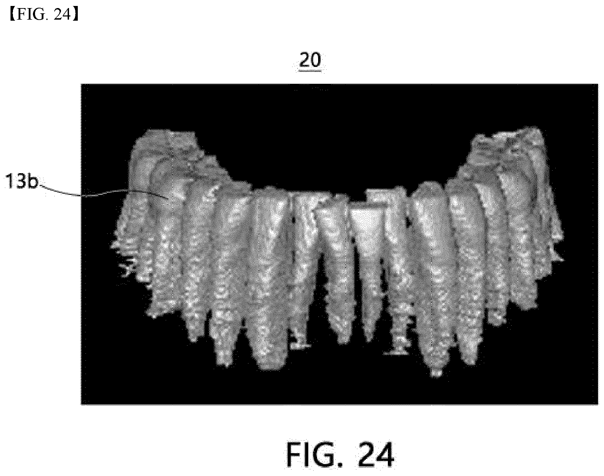

FIG. 24 is a diagram illustrating a divided tooth area in a CT image.

Referring to FIG. 24, the second tooth area 13b in the CT image 20 may be detected by using a learning model. That is, by using a deep learning method, a second learning model is generated by learning a second learning image based on Hounsfield unit (HU) values of a plurality of voxels included in the CT image 20, and by inputting the CT image 20 to the learning model, the second tooth area 11b is detected from the CT image 20 based on the second learning image.

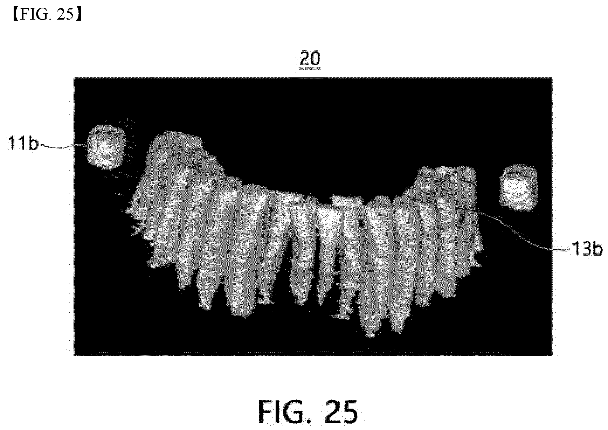

FIG. 25 is a diagram illustrating a second matching reference point in a CT image.

As illustrated in FIG. 25, a second matching reference point is generated that serves a reference for matching the oral scan image 10 and the CT image 20 by integrating the second marker 11b and the second tooth area 13b respectively divided in the CT image 10 (S40b).

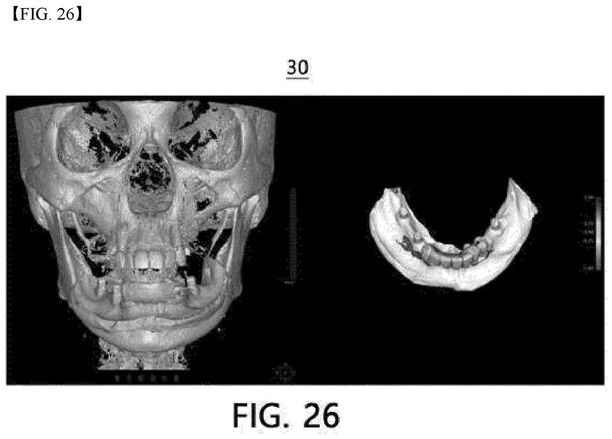

FIG. 26 is a diagram illustrating the matching of an oral scan image and a CT image by the oral image matching method according to the second example of the present invention.

Referring to FIG. 26, the first mark 11a and the first tooth area divided in the oral scan image 10 and the second marker 11b and the second tooth area divided in the CT image 20 are used as matching reference points, respectively, to automatically match the oral scan image 10 and the CT image 20 (S50b).

As described above, according to the oral image matching method of the present invention, the marker and the tooth area are divided in the oral image, and the factors that interfere with matching are removed from the marker, and the image matching is automatically performed with high accuracy by using the divided marker and the tooth area, and thus, the user's convenience is improved, and as a result, it has the effects of reducing the time required for the implant planning and improving the accuracy of the implant planning.

In the detailed description of the present invention, although specific exemplary embodiments have been described, various modifications are possible without departing from the scope of the present invention. Therefore, the scope of the present invention is not limited to the described exemplary embodiments, and should be defined by the following claims and their equivalents.

### [Industrial Applicability]

The oral image marker detection method according to the present invention and the oral image matching device and method using the same can be used in various dental treatment fields such as implant surgery.

## Claims

1. An oral image marker detection method for detecting a marker, which is the reference when an oral scan image and a CT image are matched, the method comprising the steps of:
aligning oral scan images in the horizontal plane direction;
estimating a marker candidate area from the aligned oral scan images and detecting an undivided marker from the marker candidate area; and
dividing the undivided marker to estimate a marker in the undivided marker, and estimating the area from the upper surface of the undivided marker to a certain depth in the lower direction to be the marker.

2. The oral image marker detection method of claim 1, further comprising the step of:
detecting the marker in the CT image by using the volume information of the marker.

3. The oral image marker detection method of claim 1, wherein the step of detecting the undivided marker comprises the steps of:
generating a learning model by learning a plurality of learning images to which the marker is attached;
inputting the oral scan image to the learning model; and
detecting the undivided marker in the oral scan image based on the learning image.

4. The oral image marker detection method of claim 1, wherein the step of estimating the marker comprises the step of:
estimating an area exceeding the predetermined depth in the undivided marker as an adhesive layer interposed between the marker and the gum.

5. The oral image marker detection method of claim 1, wherein the step of detecting the undivided marker is a step of obtaining the number, size and coordinate information of the undivided marker.

6. The oral image marker detection method of claim 2, wherein the step of detecting the marker in the CT image comprises the steps of:
generating a mask in a shape corresponding to the marker by using the volume information of the marker; and
searching for the marker in the CT image by using the mask.

7. The oral image marker detection method of claim 2, wherein the step of detecting the marker in the CT image is a step of obtaining the number, size and coordinate information of the marker.

8. An oral image matching method for matching an oral scan image and a CT image by using a marker, the method comprising the steps of:
aligning oral scan images in the horizontal plane direction;
estimating a marker candidate area from the aligned oral scan images and detecting an undivided marker from the marker candidate area;
dividing the undivided marker to estimate a marker in the undivided marker, and estimating the area from the upper surface of the undivided marker to a certain depth in the lower direction to be the marker;
detecting the marker in the CT image by using the volume information of the marker; and
matching the oral scan image and the CT image by using the marker.

9. An oral image matching device for matching an oral scan image and a CT image by using a marker, comprising:
an image alignment unit for aligning oral scan images in the horizontal plane direction;
an undivided market detection unit for estimating a marker candidate area from the aligned oral scan images and detecting an undivided marker from the marker candidate area;
a marker estimation unit for dividing the undivided marker to estimate a marker in the undivided marker, and estimating the area from the upper surface of the undivided marker to a certain depth in the lower direction to be the marker;
a marker detection unit for detecting the marker in the CT image by using the volume information of the marker; and
an image matching unit for matching the oral scan image and the CT image by using the marker.

10. An oral image matching method for matching an oral scan image and a CT image, comprising the steps of:
respectively dividing a first marker and a first tooth area in the oral scan image;
generating a first matching reference point by integrating the first marker and the first tooth area;
dividing a second marker and a second tooth area in the CT image;
generating a second matching reference point by integrating the second marker and the second tooth area; and
matching the oral scan image and the CT image based on the first and second matching reference points.

11. The oral image matching method of claim 10, wherein the step of dividing a first marker in the oral scan image comprises the steps of:
aligning oral scan images in the horizontal plane direction;
estimating a marker candidate area from the aligned oral scan images and detecting an undivided marker from the marker candidate area; and
dividing the undivided marker to estimate a first marker in the undivided marker, and estimating the area from the upper surface of the undivided marker to a certain depth in the lower direction to be the marker.

12. The oral image matching method of claim 10, wherein the step of dividing a first marker in the oral scan image comprises the steps of:
generating a first learning model by learning a plurality of first learning images to which the first marker is attached;
inputting the oral scan image to the first learning model; and
detecting the undivided marker in the oral scan image based on the first learning image.

13. The oral image matching method of claim 10, wherein the step of dividing a first tooth area in the oral scan image comprises the steps of:
calculating curvatures of a plurality of meshes included in the oral scan image;
calculating a boundary between the first tooth area and the gum region in the oral scan image based on the curvatures of the plurality of meshes; and
dividing the first tooth area in the oral scan image based on the boundary between the first tooth area and the gum region.

14. The oral image matching method of claim 10, wherein the step of dividing a second marker in the CT image is a step of detecting the second marker in the CT image by using the volume information of the second marker.

15. The oral image matching method of claim 10, wherein the step of dividing a second marker in the CT image comprises the steps of:
generating a mask in a shape corresponding to the second marker by using the volume information of the second marker; and
searching for the second marker in the CT image by using the mask.

16. The oral image matching method of claim 10, wherein the step of dividing a second tooth area in the CT image comprises the steps of:
generating a second learning model by learning a second learning image based on Hounsfield unit (HU) values of a plurality of voxels included in the CT image;
inputting the CT image to the second learning model; and
dividing the second tooth area in the CT image based on the second learning image.

17. An oral image matching device for matching an oral scan image and a CT image, comprising:
a first marker dividing unit for dividing a first marker in the oral scan image;
a first tooth area dividing unit for dividing a first tooth area in the oral scan image;
a first matching reference point generating unit for generating a first matching reference point by integrating the first marker and the first tooth area;
a second marker dividing unit for dividing a second marker in the CT image;
a second tooth area dividing unit for dividing a second tooth area in the CT image;
a second matching reference point generating unit for generating a second matching reference point by integrating the second marker and the second tooth area; and
an image matching unit for matching the oral scan image and the CT image based on the first and second matching reference points.
